# EUROPEAN PATENT APPLICATION

(11) **EP 2 634 181 A1**
(43) Date of publication of application: **04.09.2013**
(21) Application number: 13167765.0
(22) Date of filing: 31.03.2009
(51) Int. Cl.: C07D 307/33

(54) **Process for the production of lactones**

(62) Divisional of application: 09004697.0
(71) Applicant: Cognis IP Management GmbH, 40589 Düsseldorf (DE)
(72) Inventor: Dierker, Markus, 40593 Düsseldorf (DE); Gooßen, Lukas J., 67663 Kaiserslautern (DE); Löhl, Thorsten, 57392 Schmallenberg (DE); Ohlmann, Dominik, 40597 Düsseldorf (DE)

(57) **Abstract**

The invention is directed to a process for the lactonization of carboxylic acids, wherein unsaturated carboxylic acids comprising 10 to 24 carbon atoms are reacted in the presence of a salt of fluorinated alkane sulfonic acid at temperatures between 100 °C and 200°C.

## Description

### Technical Field

The present invention relates to a process for the production of lactones using unsaturated carboxylic acids and/or 4-hydroxyl-substituted carboxylic acids.

Gamma-lactones are widely present in plants, vegetables and fruits. They are aromatic molecules that constitute the aroma and flavour of many natural products. For example gamma-decalactone and gamma-undecalactone have a peach or apricot aroma and taste.

### State of the Art

Shepard et al. (Journal of the Americal Oil Chemists' Society, Vol 46, p. 479-481, 1968**)** as well as Showell et al. (The Journal of Organic Chemistry, Vol. 33, No. 7, 1968, p. 2697-2704**)** describe a process for the lactonization of oleic acid in the presence of aqueous 70% perchloric acid. Zhou et al. (Applied Catalysis A: General 333, 238-244, 2007**)** describe a process for the lactonization of olefinic acids using solid sulfonic acids catalysts Amberlyst-15 and Naflon SCA-13. They report good yields for 3- and 4-enoic acids, but poor yields for 5-, 6-, 9-, and 10-enoic acids, which require a double bond migration before lactonization can occur. For the lactonization of oleic acid Zhou et al. report a yield of 33%. US 2008/0108116 discloses the microbial synthesis of gamma-lactones. FR 2623499 discloses a process for the simultaneous production of stearolactone and polymers of oleic acid in the presence of methanesulfonic acid.

The aim of the present invention was to provide a new process for the production of lactones, which avoids using corrosive and hazardous materials and which at the same time provides good, preferably improved yields over processes known in the art. The process should preferably be a "one-step process". The process should preferably not yield polymers of the starting material.

### Description of the Invention

One embodiment of the invention is directed to a process for the production of lactones, wherein an unsaturated carboxylic acid and/or a 4-hydroxylsubstituted carboxylic acid with at least 7 carbon atoms is reacted in the presence of a fluorinated alkane sulfonic acid or salts thereof, preferably in the presence of a trifluoromethanesulfonic acid or salts thereof.

It has surprisingly been found that the process according to the inventions results in high yields of lactones, preferably gamma-lactones. In an embodiment of the invention, the process can be conducted without employing corrosive and hazardous materials.

The term "lactones" is a collective name for cyclic esters (oxygen heterocycles), which can be regarded as inner esters of hydroxyl carboxylic acids. The most prominent members are gamma-lactones and delta-lactones. Gamma-lactones are 5 ring members; delta-lactones are 6 ring members.

### Starting material

The unsaturated carboxylic acid and/or 4-hydroxylsubstituted carboxylic acid comprise at least 7 carbon atoms (including the C atom of the carboxy-group).

The unsaturated carboxylic acid and/or 4-hydroxylsubstituted carboxylic acid carry at least one carboxy group (monocarboxylic acid). It is within the scope of the invention that carboxylic acids carrying two or more carboxy groups are used (e.g. dicarboxylic acids or tricarboxylic acids). In a preferred embodiment of the invention the unsaturated carboxylic acid and/or 4-hydroxylsubstituted carboxylic acid carry one carboxy group (monocarboxylic acid) or two carboxy groups (dicarboxylic acid).

In a preferred embodiment of the invention the unsaturated carboxylic acid and/or 4-hydroxylsubstituted carboxylic acid according to the invention comprise 7 to 24 carbons atoms, preferably 8 to 22, more preferably 10 to 20. In a preferred embodiment of the invention, a carbon acid with 18 carbon atoms is used.

As starting materials either one or more unsaturated carboxylic acid(s) or one or more 4-hydroxylsubstituted carboxylic acid(s) comprising at least 7 carbon atoms can be used, also mixtures of one or more unsaturated carboxylic acid(s) and one ore more 4-hydroxylsubstituted carboxylic acid(s) comprising at least 7 carbon atoms can be used.

The unsaturated carboxylic acid and/or the 4-hydroxylsubstituted carboxylic acid according to the invention can be further substituted, for example with one or more groups selected from the group consisting of amino group(s), hydroxyl group(s), epoxy group(s), oxa group(s), oxo group(s), heterocyclic group(s), halides such as fluorine, chlorine, bromine or iodine, allene group(s), alkoxy group(s), aryloxy group(s), nitro group(s), acetylene group(s), acetoxy group(s), cyano group(s) and isonitrile group(s).

In a preferred embodiment of the invention the unsaturated carboxylic acid and/or the 4-hydroxylsubstituted carboxylic acid are further substituted and carry at least one or more hydroxyl group(s), and/or one or more amino-group(s), and/or one or more epoxy-group(s) and/or one or more cyclic group(s). Suitable cyclic include for example aliphatic cyclic groups, aromatic cyclic groups as well as heterocyclic groups.

### Unsaturated carboxylic acids

Suitable unsaturated carboxylic acids according to the invention are any carboxylic acids which carry at least one unsaturation and comprise at least 7 C atoms. The unsaturated carboxylic acids according to the invention comprise at least one double bond (unsaturation), they may also comprise at least two, at least three or at least four double bonds. Carboxylic acids carrying one double bond are monounsaturated carboxylic acids, carboxylic acids carrying two double bonds are di-unsaturated carboxylic acids. The double bond(s) can be either in (*Z*) or (*E*) position. The unsaturated carboxylic acids can be linear or branched carboxylic acids. In a preferred embodiment of the invention unsaturated monocarboxylic acids are used.

In a preferred embodiment of the invention linear unsaturated carboxylic acids are used.

Suitable **monounsaturated carboxylic acids** according to the invention include undecenoic acid (C11), dodecenoic acid (C12), tridecenoic acid (C13), tetradecenoic acid (C14), pentadecenoic acid (C15), hexadecenoic acid (C16), heptadecenoic acid (C17), octadecenoic acid (C18), non-adecenoic acid (C19), eicosenoic acid (C20), docosenoic acid (C22), tetracosenoic acid (C24), wherein the position of the unsaturation can be at any location within the alkyl chain and the unsaturation can be either in (*Z*) or (*E*) position. The number in brackets designates the total number of carbon atoms in the carboxylic acid.

In a preferred embodiment of the invention **linear, monounsaturated carboxylic acids** are used. In a preferred embodiment of the invention **linear, monounsaturated monocarboxylic acids** are used. In a preferred embodiment of the invention **linear, monounsaturated carboxylic acids** are used selected from the group consisting of n-undecenoic acid (C11), n-dodecenoic acid (C12), n-tridecenoic acid (C13), n-tetradecenoic acid (C14), n-pentadecenoic acid (C15), n-hexadecenoic acid (C16), n-heptadecenoic acid (C17), n-octadecenoic acid (C18), n-nonadecenoic acid (C19), n-eicosenoic acid (C20), n-docosenoic acid (C22), n-tetracosenoic acid (C24), wherein the position of the unsaturation can be at any location within the alkyl chain and the unsaturation can be either in (*Z*) or (*E*) position.
Examples of **monounsaturated, linear carboxylic acids** which can be used according to the invention comprise
- (Z)-undec-10-enoic acid
- (E)-undec-10-enoic acid
- Myristoleic acid (IUPAC: (*Z*)-Tetradec-9-enoic acid, C14:1, ω-5)
- Palmitoleic acid (IUPAC: (Z)-hexadec-9-enoic acid; 16:1, ω-7)
- Oleic acid (IUPAC: (Z)-octadec-9-enoic acid; 18:1 ω-9 )
- Elaidic acid (IPAC: (E)-octadec-9-enoic acid; C18:1, ω-9)
- Erucic acid (IUPAC (*Z*)-Docos-13-enoic acid; 22:1 ω-9)
- (Z)-Octadec-2-enoic acid (18:1)
- Nervonic acid (IUPAC: (*Z*)-Tetracos-15-enoic acid; C24:1, ω-9
- Vaccenic acid, [(*E*)-Octadec-11-ensäure; C18:1],
- Petroselinic acid [(*Z*)-6-Octadecenoic acid] C18:1
   Examples of **diunsaturated, linear carboxylic acids** which can be used according to the invention comprise
- Linoleic acid (IUPAC (*all*-*Z*)-9,12-octadecadienoic acid; 18:2 ω-6)
- *(all*-*E)*-9,12-octadecadienoic acid (18:2 ω-6 )

Examples of **triunsaturated, linear carboxylic acids** which can be used according to the invention comprise
- Alpha-linolenic acid (*all-Z*)-9,12,15-Octadecatrienoic acid; C18:3 ω-3)
- Gamma-linolenic acid (*all-Z*)-6,9,12-Octadecatrienoic acid; GLA; C18:3, ω-6)
- Eleostearic acids, (Octadeca-9,11,13-trienoic acid; C18:3) such as alpha-Eleostearic acid [(9*Z*,11 *E*,13*E*)-9,11,13-Octadeca-9,11,13-trienoic oacid]
- Dihomo- gamma-linolenic acid [(*all-Z*)-8,11,14-Eicosatrienoic acid, C20:3)
   Examples of **tetraunsaturated, linear carboxylic acids** which can be used according to the invention comprise
- Arachidonic acid (IUPAC: *all*-*(Z)*-5,8,11,14-eicosatetraenoic acid. C 20:4, ω-6)
- Stearidonic acid (IUPAC: all-(*Z*)-6,9,12,15-octatetraenoid acid, C18:4, ω-3)
   Examples of **pentaunsaturated, linear carboxylic acids** which can be used according to the invention comprise
- clupa(no)donic acid [(*all-Z*)-4,8,12,15,19-docosapentaenoic acid, C 22:5]
- eicosapentaenoic acid [(*all-Z*)-5,8,11,14,17-lcosapentaenoid acid, EPA, C22:5]
Examples of **hexaunsaturated, linear carboxylic acids** which can be used according to the invention comprise
- Docosahexaenoic acid [(4Z,7Z,10Z,13Z,16Z,19Z)-docosa-4,7,10,13,16,19-hexaenoic acid, C 22:6, cervonic acid, ω-3)

Examples of suitable unsaturated carboxylic acids carrying a cyclic group are furan carboxylic acids, so called F-fatty acids; further examples are carboxylic acids carrying a cyclopropene group.

Suitable unsaturated carboxylic acids according to the invention are carboxylic acids according to formula (I) H-(CH₂)ₘ-CH=CH-(CH₂)ₙ-COOH,
- wherein n is a number from 0 to 24, preferably n is equal or greater than 3
- wherein m is a number from 0 to 18.

In a preferred embodiment of the invention, unsaturated carboxylic acids according to formula (I) are used, wherein m+n ≤ 21.

In a preferred embodiment of the invention an unsaturated carboxylic acid is used selected from the group consisting of palmitoleic acid (IUPAC: (Z)-hexadec-9-enoic acid; 16:1, ω-7), Oleic acid (IUPAC: (Z)-octadec-9-enoic acid; 18:1 ω-9 ), Elaidic acid (IUPAC: (E)-octadec-9-enoic acid, C18:1, ω-9) (Z)-Octadec-2-enoic acid (18:1), Vaccenic acid, [(*E*)-Octadec-11-ensäure, C18:1] and petroselinic acid [(*Z*)-6-Octadecenoic acid, C18:1]. In a preferred embodiment of the invention oleic acid is used.

### 4 -Hydroxylsubstituted carboxylic acids

Suitable 4-Hydroxylsubstituted carboxylic acids according to the invention are any carboxylic acids which carry a Hydroxyl-group at position 4 (in relation to the carboxy-group) and comprise at least 7 C atoms. The 4-Hydroxylsubstituted carboxylic acids can be saturated or unsaturated. In a preferred embodiment of the invention 4-Hydroxylsubstituted monocarboxylic acids are used.

Suitable 4-Hydroxylsubsituted carboxylic acids can be selected from the group consisting of
(II) R₁-CH(OH)-CH₂-CH₂-COOH and/or
(III) R₁-CH(OH)-CHR₂-CHR₃-COOH and/or
(IV) R₁-CH(OH)-CH=CH-COOH and/or
   - wherein R₁ is a linear or branched, saturated or unsaturated alkyl group with 3 to 20 C atoms
   - wherein R₂ and R₃ are independently of each other -H or a linear or branched, saturated or unsaturated alkyl group with 1 to 6 alkyl groups.

In a preferred embodiment of the invention 4-Hydroxylsubstituted carboxylic acids according to formula (II) are used. In a preferred embodiment of the invention linear 4-Hydroxylsubstitued carboxylic acids are used.

Examples of suitable 4-Hydroxylsubstitued carboxylic acids are 4-Hydroxyl-n-dodecanoic acid (= 4-Hydroxyl lauric acid), 4-Hydroxyl-n-tetradecanoic acid (= 4-Hydroxyl myristic acid), 4-Hydroxyl-n-hexadecanoic acid (= 4-Hydroxyl palmitic acid) and 4-Hydroxyl-n-octadecanoic acid (= 4-Hydroxyl stearic acid).

It is within the scope of the invention that unsaturated carboxylic acids and/or 4-hydroxylsubstituted carboxylics acids with at least 7 carbon atoms can also be used in an esterified form. The carboxylic acid is then released in a first reaction step (saponification) preceding the process according to the invention. Suitable esterified forms that can be employed are for example alkylesters of the carboxylic acids, wherein the alkyl moiety carries 1 to 6 C atoms, e.g. methyl or butyl esters or esters of the carboxylic acid with polyols such as e.g. glycerol (e.g. mono-, di-, triglycerids).

### Fluorinated alkane sulfonic acid or salt thereof and/or a metal salt comprising a non-coordinating anion

The process according to the invention can be conducted in the presence of a fluorinated alkane sulfonic acid or a salt thereof (preferably trifluoromethanesulfonic acid or a salt thereof) and/or in the presence of a metal salt comprising a non-coordinating anion.

Thus one embodiment of the invention is directed to a process for the production of lactones, wherein unsaturated carboxylic acids and/or 4-hydroxylsubstituted carboxylic acids with at least 7 carbon atoms are reacted in the presence of a fluorinated alkane sulfonic acid or a salt thereof (preferably trifluoromethanesulfonic acid or a salt thereof) and/or in the presence of a metal salt comprising a non-coordinating anion.

In a preferred embodiment of the invention the fluorinated alkane sulfonic acid or salts thereof (preferably trifluoromethanesulfonic acid or salt thereof) and/or in the metal salt comprising a non-coordinating anion is (are) present in an amount of less than 300 mol%, preferably less than 100 mol%, more preferably less than 50mol%. Even more preferred these substances are present in an amount of less than 20mol%, preferably less than 15 mol%.

Suitable fluorinated alkane sulfonic acids can be partially fluorinated alkane sulfonic acids or completely fluorinated alkane sulfonic acids (= perfluoro alkane sulfonic acids).

Suitable fluorinated alkane sulfonic acids are for example perfluoro alkane sulfonic acids of the general formula R_{f}-SO₃H, wherein R_{f} is selected from the group consisting of CF₃, C₄F₉ and C₈F₁₇. Thus suitable alkane sulfonic acids are trifluoromethanesulfonic acid, nonafluorobutane sulfonic acid and perfluoro octane sulfonic acid.

In a preferred embodiment of the invention trifluoromethanesulfonic acid and salts thereof and/or nonafluoro butane sulfonic acid and salts thereof are used.

In principle any salt of the fluorinated alkane sulfonic acid can be used. Suitable salts are salts of metallic cations, salts of organometallic cations or salts of non-metallic cations. Suitable salts can be selected from the group consisting of Lithium(I), Sodium(I), Potassium(I), Magnesium(II), Calcium(II), Barium(II), Scandium(III), Yttrium(III), Lanthanum(II), Cerium(III), Praseodymium(III), Neodymium(III), Samarium(III), Europium(III), Gadolinium(III), Terbium(III), Dysprosium(III), Holmium(III), Thulium(III), Ytterbium(III), Hafnium(IV), Iron(II), Nickel(II), Copper(I), Copper(II), Silver(I), Zinc(II), Mercury(II), Aluminum(III), Indium(III), Tin(II) and Bismuth(III).

Further suitable cations are selected from the group consisting of
- 2,6-Diisopropylphenylimidoneophylidenemolybdenum(VI)
- (+)-1,2-Bis((2S,5S)-2,5-diethylphospholano)bis(1,5-cyclooctadiene)rhodium(I)
- benzene(cyclooctadiene)rhodium(I)
- Tris(acetonitrile)pentamethylcyclopentadienylruthenium(II)
- Tri-n-butyltin(IV)
- Triphenylphosphinegold(I)

### Trifluoromethanesulfonic acid or salts thereof

The process according to the invention is preferably conducted in the presence of trifluoromethanesulfonic acid or a salt thereof. In a preferred embodiment of the invention a salt of trifluoromethanesulfonic acid is used.

In principle any salt of trifluoromethanesulfonic acid can be used. Suitable salts are salts of metallic cations, salts of organometallic cations or salts of non-metallic cations. Suitable salts can be selected from the group consisting of Lithium(I), Sodium(I), Potassium(I), Magnesium(II), Calcium(II), Barium(II), Scandium(III), Yttrium(III), Lanthanum(II), Cerium(III), Praseodymium(III), Neodymium(III), Samarium(III), Europium(III), Gadolinium(III), Terbium(III), Dysprosium(III), Holmium(III), Thulium(III), Ytterbium(III), Hafnium(IV), Iron(II), Nickel(II), Copper(I), Copper(II), Silver(I), Zinc(II), Mercury(II), Aluminum(III), Indium(III), Tin(II) and Bismuth(III).

In a preferred embodiment of the invention the salts of trifluoromethanesulfonic acid are selected from the group consisting of the salts of Copper(I), Copper(II), Indium(III), Scandium(III), Ytterbium(III), Silver(I) and Bismuth(III).

In a most preferred embodiment of the invention a silver salt of trifluoromethanesulfonic acid is used.

Further suitable cations are selected from the group consisting of
- 2,6-Diisopropylphenylimidoneophylidenemolybdenum(VI)
- (+)-1,2-Bis((2S,5S)-2,5-diethylphospholano)bis(1,5-cyclooctadiene)rhodium(I)
- benzene(cyclooctadiene)rhodium(I)
- Tris(acetonitrile)pentamethylcyclopentadienylruthenium(II)
- Tri-n-butyltin(IV)
- Triphenylphosphinegold(I)

These salts are commercially available, for example from ABCR, Acros Organics or Strem.

It is within the scope of the invention that the salts of fluorinated alkane sulfonic acids can be generated in situ, e.g. the salt of trifluoromethanesulfonic acid can be generated in situ, for example by using trifluoromethanesulfonic acid together with a source for the metal, e.g. a Silver (II) oxide or a silver (III) oxid, a copper (II) oxide or a copper (III) oxide as well as silver chloride and/or silver iodide.

It is within the scope of the invention that one or more salts of trifluoromethanesulfonic acid can be used. Trifluoromethanesulfonic acid or the salts thereof can be used in a range of 2 to 20, preferably 5 to 15 mol%.

### Metal salt comprising a non-coordinating anion

The process according to the invention can be conducted in the presence of a metal salt comprising a non-coordinating anion. Non-coordinating anions are also sometimes referred to as weakly coordinating anions (WCA).

Suitable non-coordinating anions are those described by I. Krossing and I. Raabe in Angew. Chem Int. Ed, 2004, 43, 2066 - 2090.

Especially preferred non-coordinating anions are those described in section 2.1 to section 2.5 by I. Krossing and I. Raabe in Angew. Chem Int. Ed., 2004, 43, pages 2066 - 2072.

Suitable non-coordinating anions are for example
- sulfate
- nitrate
- MF₆⁻, wherein M = P (phosphor), As (arsenic) or Sb (antimony), such as hexafluorophosphate, hexafluoroantimonate.
- perchlorate [ClO₄⁻]
- tetrafluoroborate [BF₄⁻]
- tetraarylborates, such as tetraphenylborate [BPh₄⁻], or tetraarylborates wherein the aryl group can be further substituted, e.g. with fluorine, such as [B(Ar^{F})₄⁻], wherein Ar^{F} = C₆H₅-3,5(CF₃)₂)
- tetrahalogenoaluminate [AlX₄⁻, wherein X can be Cl, Br, I or F], such as tetrachloroaluminate, tetrafluoroaluminate, tetraiodoaluminate or tetrabromoaluminate
- Carborane-based anions such as tetracarboranes
- bis(trifluoromethane) sulphonamide
- perfluoro-tert-butylate.

Preferred tetraarylborates are those listed in section 2.1 on page 2067 to page 2069 of Angew. Chem Int. Ed, 2004, 43. Preferred carborane-based anions are those listed in section 2.2 on page 2069 of Angew. Chem Int. Ed, 2004, 43.

In a preferred embodiment of the invention the non-coordinating anion is selected from the group consisting of sulphate; nitrate; MF₆⁻, wherein M = P (phosphor), As (arsenic) or Sb (antimony), such as hexafluorophosphate, hexafluoroantimonate; perchlorate [ClO₄⁻]; tetrafluoroborate, [BF₄⁻]; tetraarylborates, such as tetraphenlyborate [BPh₄⁻], or tetraarylborates wherein the aryl group can be further substituted, e.g. with fluorine, such as [B(Ar^{F})₄⁻], wherein Ar^{F} = C₆H₅-3,5(CF₃)₂); tetrahalogenaluminate [AlX₄⁻, wherein X can be Cl, Br, I or F], such as tetrachloroaluminate, tetrafluoroaluminate, tetraiodoaluminate or tetrabromoaluminate; Carborane-based anions such as tetracarboranes; bis(trifluoromethane) sulphonamide and perfluoro-tert-butylate.

Suitable metal salts are selected from the group consisting of Lithium(I), Sodium(I), Potassium(I), Magnesium(II), Calcium(II), Barium(II), Scandium(III), Yttrium(III), Lanthanum(II), Cerium(III), Praseodymium(III), Neodymium(III), Samarium(III), Europium(III), Gadolinium(III), Terbium(III), Dysprosium(III), Holmium(III), Thulium(III), Ytterbium(III), Hafnium(IV), Iron(II), Nickel(II), Copper(I), Copper(II), Silver(I), Zinc(II), Mercury(II), Aluminum(III), Indium(III), Tin(II) and Bismuth(III).

In a preferred embodiment of the invention the salts are selected from the group consisting of the salts of Copper(I), Copper(II), Indium(III), Scandium(III), Ytterbium(III), Silver(I) and Bismuth(III).

In one embodiment the invention is directed to a process for the production of lactones, wherein unsaturated carboxylic acids and/or 4-hydroxylsubstituted carboxylic acids with at least 7 carbon atoms are reacted in the presence of a metal salt comprising a non-coordinating anion, wherein the non-coordinating anion is selected from the group consisting of sulphate; nitrate; MF₆⁻, wherein M = P (phosphor), As (arsenic) or Sb (antimony), such as hexafluorophosphate, hexafluoroantimonate; perchlorate [ClO₄⁻]; tetrafluoroborate, [BF₄⁻]; tetraarylborates, such as tetraphenlyborate [BPh₄⁻], or tetraarylborates wherein the aryl group can be further substituted, e.g. with fluorine, such as [B(Ar^{F})₄⁻], wherein Ar^{F} = C₆H₅-3,5(CF₃)₂); tetrahalogenaluminate [AlX₄⁻, wherein X can be Cl, Br, I or F], such as tetrachloroaluminate, tetrafluorooaluminate, tetraiodoaluminate or tetrabromoaluminate; Carborane-based anions such as tetracarboranes; bis(trifluoromethane) sulphonamide and perfluoro-tert-butylate.

In one embodiment the invention is directed to a process for the production of lactones, wherein unsaturated carboxylic acids and/or 4-hydroxylsubstituted carboxylic acids with at least 7 carbon atoms are reacted in the presence of a metal salt comprising a non-coordinating anion, wherein the non-coordinating anion is selected from the group consisting of sulphate; nitrate; MF₆⁻, wherein M = P (phosphor), As (arsenic) or Sb (antimony), such as hexafluorophosphate, hexafluoroantimonate; perchlorate [ClO₄⁻]; tetrafluoroborate, [BF₄⁻]; tetraarylborates, such as tetraphenlyborate [BPh₄⁻], or tetraarylborates wherein the aryl group can be further substituted, e.g. with fluorine, such as [B(Ar^{F})₄⁻], wherein Ar^{F} = C₆H₅-3,5(CF₃)₂); tetrahalogenaluminate [AlX₄⁻, wherein X can be Cl, Br, I or F], such as tetrachloroaluminate, tetrafluoroaluminate, tetraiodoaluminate or tetrabromoaluminate; Carborane-based anions such as tetracarboranes; bis(trifluoromethane) sulphonamide and perfluoro-tert-butylate and wherein the metal salt is selected from the group consisting of Copper(I), Copper(II), Indium(III), Scandium(III), Ytterbium(III), Silver(I) and Bismuth(III).

The metal salt comprising a non-coordinating anion can be preferably used in a range of 2 to 20, more preferably 5 to 15 mol%.

### Process

The process according to the invention can be conducted at temperatures between 20 and 200 °C, preferably between 80 and 180 °C, preferably between 100 and 170 °C, most preferably between 120 and 160 °C, most preferably between 130 and 135 °C.

The process according to the invention can be conducted without a solvent (solvent free) or in the presence of a solvent. In case a solvent is used any common organic solvents can be employed as long as the boiling point is compatible with the reaction temperature selected. Examples for suitable solvents are dimethylacetamide (DMA), dimethylformamide (DMF), alcohols (such as isopropanol, n-butanol), pyridine, dimethyl sulfoxide (DMSO), tetrahydrofuran (THF), 1-Methyl-2-pyrrolidinon (NMP), 1,4-dioxane, acetonitrile, acetone, hydrocarbons, aromatic hydrocarbons, such as benzene, ethylbenzene, toluene and xylenes (ortho-xylene, m-xylene, p-xylene) as well as halogenated (aromatic) hydrocarbons, such as 1,2- dichlorethan or fluorohydrocarbons, chlorinated hydrocarbons and brominated hydrocarbons.

In a preferred embodiment of the invention halogenated hydrocarbons, preferably halogenated aromatic hydrocarbons are used as solvents. In a preferred embodiment of the invention chlorinated aromatic hydrocarbons are used as solvents. A suitable solvent is chlorobenzene, preferably a mixture of chlorobenzene and any dichlorobenzene (such as o-dichlorobenzene or p-dichlorobenzene or m-chlorobenzene and mixtures thereof) is used.

### Products

The process according to the invention results in lactones. Lactones obtained are mainly gamma-lactones and/or delta-lactones, preferably gamma-lactones are obtained.

In a preferred embodiment of the invention the process is directed to a process for the production of gamma-lactones, whereby unsaturated carboxylic acids and/or 4-hydroxylsubstituted carboxylics acids with at least 7 carbon atoms are reacted in the presence of a trifluoromethanesulfonic acid or salts thereof.

Lactonization of oleic acid will result in the gamma lactone 4-tetradecyl-5-butyrolactone (gamma-stearolactone, gamma-octadecalactone).

One embodiment of the invention is directed to a process for the production of 4-tetra-decyl-5-butyrolactone, whereby oleic acid is reacted in the presence of a silver salt of trifluoromethanesulfonic acid.

### Examples

All solvents and reagents were purified by standard techniques. ¹H and ¹³C NMR spectra were recorded at room temperature in CDCl₃ solvent on Bruker DPX 400 or Bruker Avance 600 NMR spectrometers. Chemical shifts are reported in ppm with respect to residue solvent protons (7.25 and 77.0 ppm for CDCl₃). Coupling constants (J) are quoted in Hz. Mass spectra were obtained on a Varian Saturn 2100 T mass spectrometer operating at 70 eV using EI ionization.

### General procedure: lactonization of fatty acids catalyzed by silver trifluoromethane sulfonate:

A flame-dried 20 mL headspace vial with stirring bar was charged with silver trifluoromethane sulfonate (38.5 mg, 0.15 mmol), sealed with a Teflon septum and purged with nitrogen. *Via* syringe, the fatty acid (1.0 mmol) and chlorobenzene (2 mL) were added and the reaction mixture was heated to 130 °C for 20 h. After cooling to room temperature, the solvent was removed in *vacuo* and the product was purified by flash column chromatography on silica gel (diethyl ether - hexane).

### Example 1. Lactonization of oleic acid

γ**-Octadecalactone (2a)** was synthesized from oleic acid [(9Z)-Octadec-9-enoic acid] (282 mg, 1.0 mmol) following the general procedure. **2a** was obtained after column chromatography as colorless solid (143 mg, 51%). CAS-Nr. 502-26-1. *R*_{f} = 0.59 (ethyl actetate - hexane 1:2). ¹H-NMR (400 MHz, CDCl₃): δ = 4.40-4.50 (m, 1H), 2.44-2.54 (m, 2H), 2.29 (dd, *J* = 12.9, 6.5 Hz, 1H), 1.76-1.87 (m, 1H), 1.64-1.75 (m, 1H), 1.49-1.61 (m, 1H), 1.42 (d, *J* = 6.7 Hz, 1H), 1.38 (s, 2H), 1.23 (s, 21H), 0.79-0.88 (m, 3H). ¹³C-NMR (100 MHz, CDCl₃): δ = 177.0, 80.9, 35.6, 31.9, 29.6, 29.5, 29.4, 29.3, 28.8, 28.0, 25.2, 22.6, 14.0. MS (EI, 70 eV): m/z (%) = 283(59), 264(40), 246(60), 220(38), 134(38), 85(100), 69(68).

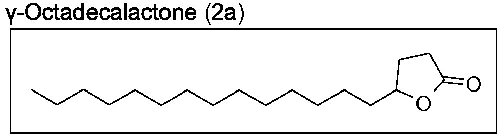

### Example 2: Lactonization of oleic acid: Multi-gram scale

A flame-dried 1 L round-bottom flask with stopcock, stirring bar and reflux condenser was charged with chlorobenzene (70 mL) and o-dichlorobenzene (420 mL). Silver trifluoromethane sulfonate (15.0 g, 57.2 mmol) and ca. 95 % oleic acid [(9Z)-Octadec-9-enoic acid] (180 g, 572 mmol) were added under nitrogen atmosphere and the mixture was heated to reflux (oil ca. 175 °C) for 16 h. After cooling to room temperature and filtering through celite, solvent was removed *in vacuo* (rotavap, 0.01 mbar, 80 °C) with additions of toluene to facilitate evaporation. The crude product was dissolved in hot ethanol and left to cool down to 20 °C, then 4 °C. The light brown precipitate was collected by filtration and recrystallized from ethanol, until NMR showed a purity >85%. The mother liquors were concentrated and left for crystallization at 4 °C to obtain more product. The lactone **2a** was obtained as light-brown solid in an overall yield of ca. 50%.

### Example 3: Lactonization of 10-undecenoid acid

γ**-Undecalactone (2b)** was synthesized from 10-undecenoic acid (184 mg, 1.0 mmol) following the general procedure. **2b** was obtained after column chromatography as light yellow liquid (131 mg, 71%). CAS-Nr. 104-67-6. *R*_{f} = 0.25 (diethyl ether - hexane 1:1). ¹H-NMR (600 MHz, CDCl₃): δ = 4.40-4.45 (m, 1H), 2.47 (dd, *J* = 9.5, 6.9 Hz, 2H), 2.27 (td, *J* = 13.2, 6.9 Hz, 1H), 1.76-1.83 (m, 1H), 1.64-1.71 (m, 1H), 1.51-1.57 (m, 1H), 1.36-1.43 (m, 1H), 1.29-1.34 (m, 1H), 1.20-1.27 (m, 8H), 0.82 (t, *J* = 7.0 Hz, 3H). ¹³C-NMR (150 MHz, CDCl₃): δ = 177.2, 80.9, 35.4, 31.6, 29.1, 29.0, 28.7, 27.9, 25.1, 22.5, 13.9. MS (EI, 70 eV): m/z (%) = 185(22), 128(19), 95(19), 85(100), 57(31), 41(28). 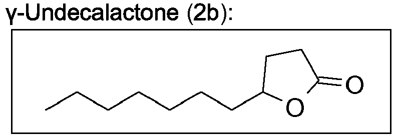
1. Process for the production of lactones, wherein unsaturated carboxylic acids and/or 4-hydroxylsubstituted carboxylic acids with at least 7 carbon atoms are reacted in the presence of a fluorinated alkane sulfonic acid or salts thereof.
2. Process according to claim 1, wherein trifluoromethanesulfonic acid or salts thereof, preferably a salt of trifluoromethanesulfonic acid is used.
3. Process according to at least one of the preceding claims, wherein the salt of trifluoromethanesulfonic acid is selected from the group consisting of Copper(I), Copper(II), Indium(III), Scandium(III), Ytterbium(III), Silver(I) and Bismuth(III) salts.
4. Process according to at least one of the preceding claims, wherein a silver salt of trifluoromethanesulfonic acid is used.
5. Process according to at least one of the preceding claims, wherein the carboxylic acid comprises 7 to 24 carbons atoms.
6. Process according to at least one of the preceding claims, wherein the carboxylic acid is a linear carboxylic acid.
7. Process according to at least one of the preceding claims, wherein the unsaturated carboxylic acid is a monounsaturated carboxylic acid.
8. Process according to at least one of the preceding claims, wherein an unsaturated carboxylic acid according to formula (I) is used

   (I) H-(CH₂)ₘ-CH=CH-(CH₂)ₙ-COOH,

   wherein n is equal to or greater than 3, m is 0 to 18.
9. Process according to claim 8, wherein in formula (I) m+n ≤ 21.
10. Process according to any preceding claim, wherein oleic acid is used as the unsaturated carboxylic acid.
11. Process according to at least one of the preceding claims, wherein the reaction is conducted at temperatures between 80 and 180 °C, preferably between 100 and 170 °C, most preferably between 120 and 160 °C, most preferably between 130 and 135 °C.
12. Process according to at least one of the preceding claims, wherein the reaction is conducted in the presence of a solvent, preferably in the presence of a solvent selected from the group of halogenated hydrocarbons.
13. Process according to at least one of the preceding claims, wherein the trifluoromethanesulfonic acid or salts thereof are present in a range of 2 to 20, preferably 5 to 15 mol%.

## Claims

1. Process for the production of gamma-lactones, wherein unsaturated carboxylic acids comprising 10 to 24 carbon atoms are reacted in the presence of a salt of a fluorinated alkane sulfonic acid, wherein the reaction is conducted at temperatures between 100°C and 200 °C.

2. Process according to claim 1, wherein a salt of trifluoromethanesulfonic acid is used.

3. Process according to at least one of the preceding claims, wherein the salt of trifluoromethanesulfonic acid is selected from the group consisting of Copper(I), Copper(II), Indium(III), Scandium(III), Ytterbium(III), Silver(I) and Bismuth(III) salts.

4. Process according to at least one of the preceding claims, wherein a silver salt of trifluoromethanesulfonic acid is used.

5. Process according to at least one of the preceding claims, wherein the carboxylic acid is a linear carboxylic acid.

6. Process according to at least one of the preceding claims, wherein an unsaturated carboxylic acid according to formula (I) is used
(I) H-(CH₂)ₘ-CH=CH-(CH₂)ₙ-COOH,
wherein n is equal to or greater than 3, m is 0 to 18.

7. Process according to claim 6, wherein in formula (I) m+n ≤ 21.

8. Process according to any preceding claim, wherein oleic acid is used as the unsaturated carboxylic acid.

9. Process according to at least one of the preceding claims, wherein the reaction is conducted in the presence of a solvent, preferably in the presence of a solvent selected from the group of halogenated hydrocarbons.

10. Process according to at least one of the preceding claims, wherein the salts of the trifluoromethanesulfonic acid are present in a range of 2 to 20, preferably 5 to 15 mol%.
